# EUROPEAN PATENT APPLICATION

(11) **EP 4 818 309 A1**
(43) Date of publication of application: **07.10.2026**
(21) Application number: 24898263.9
(22) Date of filing: 02.12.2024
(51) Int. Cl.: C12N 5/071, G01N 33/50, A61K 35/36, A61P 17/00

(54) **NOVEL METHOD FOR PREPARING SKIN ORGANOID**

(30) Priority: 30.11.2023 KR 20230171906
(71) Applicant: Organoidsciences Ltd., Seongnam-si, Gyeonggi-do 13493 (KR)
(72) Inventor: WULANSARI, Noviana, Seongnam-si, Gyeonggi-do 13493 (KR); KIM, Hyun-Mun, Seongnam-si, Gyeonggi-do 13493 (KR); LIM, Su Jin, Seongnam-si, Gyeonggi-do 13493 (KR); KIM, Yong Il, Seongnam-si, Gyeonggi-do 13493 (KR); LEE, Kun Gu, Seongnam-si, Gyeonggi-do 13493 (KR)
(74) Representative: Rückerl, Florian
(86) International application number: PCT/KR2024/019510
(87) International publication number: WO 2025/116671

(57) **Abstract**

The present invention relates to a method for preparing a skin organoid. In addition, the present invention relates to: a skin organoid prepared by the preparation method; a preparation for preventing or treating skin diseases, the preparation comprising the skin organoid; a method for evaluating the efficacy of a substance for preventing or treating skin diseases using the skin organoid; and a method for screening such a substance. The skin organoid prepared according to the present invention has a high degree of similarity with human skin tissue, may include hair follicles, and can regenerate damaged skin tissue when implanted into a living body. Therefore, the method for preparing a skin organoid according to the present invention can be used to prepare a skin organoid having a similar morphology and function to living skin tissue, and the prepared skin organoid can be used as a regenerative therapeutic agent for skin diseases, or can be advantageously used in a method for evaluating the efficacy of or screening a therapeutic agent for skin diseases.

## Description

### [Technical Field]

The present invention relates to a novel method for preparing a skin organoid. In addition, the present invention relates to: a skin organoid prepared by the method; an organoid composition capable of regenerating damaged skin using the skin organoid; and a method for evaluating efficacy of and a method for screening a therapeutic agent for a skin disease using the skin organoid.

### [Background Art]

Skin diseases caused by skin damage or aging have been treated by chemical peeling, laser irradiation, drug administration, or the like; however, such treatments have disadvantages such as pain, scarring, and side effects. Recently, skin organoids have emerged as a new strategy for treating skin diseases capable of overcoming such disadvantages, and numerous studies thereon have been conducted. Organoids are aggregates of cells differentiated from specific stem cells, and may be used for regeneration of damaged tissues because they have structural and functional characteristics highly similar to those of actual tissues in the body.

In addition, organoids can mimic the structure and function of specific tissues, and thus are highly expected in the art because they may be used for evaluating efficacy of or screening therapeutic agents as alternatives to cell and animal experiments.

However, since skin inherently has a multilayered structure, there are many limitations in preparing skin organoids compared to other types of organoids.

### [Technical Problem]

Accordingly, the present inventors conducted various studies to mimic skin organoids having a multilayered structure in terms of the structure and function of skin. As a result, the present inventors developed a method for preparing skin organoids using a concentration gradient of a specific culture component. The skin organoids prepared by the method are similar to actual skin tissues in structure and function, and may comprise hair follicles. The present inventors experimentally demonstrated that such prepared skin organoids can regenerate damaged skin tissues when transplanted into a living body, and can be usefully employed for evaluating efficacy of or screening therapeutic agents for skin diseases, thereby completing the present invention.

### [Technical Solution]

Each description and embodiment disclosed in the present invention may also be applied to other descriptions and embodiments. That is, all combinations of various elements disclosed in the present invention fall within the scope of the present invention. In addition, the scope of the present invention is not limited by the specific descriptions provided below.

In addition, terms not specifically defined herein should be understood to have meanings commonly used in the technical field to which the present invention pertains. Unless specifically defined otherwise by the context, singular forms include plural forms, and plural forms include singular forms.

One aspect of the present invention provides a method for preparing a skin organoid, comprising:
a) differentiating pluripotent stem cells into ectoderm by culturing the cells in a first medium; and
b) differentiating a product of step a) into cranial neural crest cells by culturing the product in a second medium.

Alternatively, the preparation method may also be described as comprising:
a) culturing pluripotent stem cells in a first medium to differentiate into surface ectoderm; and
b) culturing a product of step a) in a second medium to differentiate into cranial neural crest cells.

As used herein, the term "organoid" refers to a cell aggregate having a three-dimensional structure. It is defined as a miniature and simplified version of an organ, which is prepared through an artificial culture process without being collected, obtained, or isolated from an animal or the like.

Step a) is a step of culturing pluripotent stem cells in a first medium. Here, differentiation into surface ectoderm may be induced.

As used herein, the term "stem cell" refers to a cell having pluripotency capable of differentiating into endodermal, mesodermal, and ectodermal cells, or multipotency capable of differentiating into cells closely associated with a tissue or function. The ectoderm may differentiate into epidermis, glandular tissue, neuroepithelium, or nervous system. It may differentiate into an epidermal layer in the skin organoid of the present invention.

As used herein, the term "differentiation" refers to specialization of a cell such that the cell acquires a form and function suitable for its respective role during cell division and proliferation. For example, it may be a process in which pluripotent stem cells differentiate into ectoderm or ectoderm differentiates into an epidermal layer.

As used herein, the term "pluripotent stem cell" refers to a cell having differentiation potency capable of differentiating into all cells constituting an individual and self-renewal ability, and may be used interchangeably with the term "multipotent stem cell". The pluripotent stem cell may be, for example, an embryonic stem cell, an embryonic germ cell, or an induced pluripotent stem cell, but is not limited thereto. Preferably, the pluripotent stem cell is an embryonic stem cell or an induced pluripotent stem cell. The term "induced pluripotent stem cell" may be used interchangeably with the term "dedifferentiated stem cell" or "artificial pluripotent stem cell".

In the present invention, step b) is a step of culturing the material obtained in step a) in a second medium. Here, differentiation into cranial neural crest cells may be induced.

As used herein, the term "cranial neural crest cell" refers to a cell that differentiates into mesenchymal cells and neuro-glial cells and then forms a dermal layer.

As used herein, the term "dermal layer" refers to a thick cell layer located beneath the epidermal layer and constituting about 90% of the skin. The dermal layer is known to be involved in skin elasticity and gloss, to supply nutrients to the epidermis, and to maintain and protect other tissues of the skin.

As used herein, the term "subcutaneous fat layer" refers to a tissue located at the lowermost part of the skin, and may be used interchangeably with the term "subcutaneous tissue layer". Adipocytes in the subcutaneous fat layer are known to produce subcutaneous fat and to function in nutrient storage, regulation of body temperature and moisture, maintenance of skin elasticity, or mitigation of external impact.

The first medium and the second medium may comprise bone morphogenetic protein (BMP). The BMP may be, for example, a BMP2 protein, BMP3 protein, BMP4 protein, BMP6 protein, BMP7 protein, BMP8 protein, BMP9 protein, BMP10 protein, BMP11 protein, or BMP15 protein, but is not limited thereto.

Organoids are characterized in that preparation results vary significantly depending on culture conditions. Examples of the culture conditions include culture period, culture temperature, culture medium, and culture substrate, which are important factors determining characteristics of organoids. Among these, medium conditions play the greatest role in growing organoids to have characteristics similar to desired tissues or organs by regulating various signaling pathways in stem cells. Since organoids cultured under different medium conditions exhibit greatly different characteristics in terms of similarity to actual tissues or viability, the present invention established optimal culture medium conditions capable of obtaining a sufficient number of organoids having morphology and function highly similar to those of actual tissues and applicable to therapeutic use or screening of therapeutic agents.

Specifically, in the method for preparing a skin organoid according to the present invention, the concentration of BMP in the second medium is lower than the concentration of BMP in the first medium. For example, the BMP concentration in the first medium may be 10 ng/mL or more, 15 ng/mL or more, 20 ng/mL or more, or 25 ng/mL or more, and the BMP concentration in the second medium may be 10 ng/mL or less, 5 ng/mL or less, 2.5 ng/mL or less, or 1 ng/mL or less.

In preparation of the skin organoid, when a decreasing concentration gradient is formed by reducing the BMP concentration in step b) compared to step a), various advantages may be obtained in induction of cranial neural crest cells, melanocytes, hair follicles, and the like, compared to when the BMP concentrations in steps a) and b) are the same as or similar to each other. As a result, the skin organoid prepared according to the preparation method of the present invention can structurally and functionally mimic actual skin and, for example, mimic a multilayered structure or comprise hair follicles.

As used herein, the term "melanocyte" refers to a cell present in the epidermis, dermis, or mucosal epithelium of the skin and derived from cranial neural crest cells, and is known to be involved in determining the color of skin or hair.

As used herein, the term "hair follicle" refers to a sac surrounding hair and formed across the epidermis and dermis, and is known to protect a hair root, which is a portion of hair located within the skin, and to supply nutrients to the hair root to allow hair growth.

In addition, the first medium and the second medium may further comprise a transforming growth factor β (TGFβ) inhibitor. The TGFβ inhibitor may be, for example, A83-01, SB-431542, SB-505124, SB-525334, SD-208, LY-364947, or SJN-2511, but is not limited thereto.

In addition, the skin organoid may comprise a multilayered structure including an epidermal layer, a dermal layer, and a subcutaneous tissue layer, or may consist of the multilayered structure. In addition, the skin organoid may comprise a hair follicle.

The preparation method according to the present invention may further comprise step c) of seeding pluripotent stem cells in a third medium prior to step a).

The preparation method according to the present invention may further comprise step d) of inducing caudalization in the material obtained in step b) using a fourth medium. The fourth medium may comprise a BMP inhibitor and/or a mitogenic growth factor. The BMP inhibitor may be, for example, dorsomorphin, LDN-193189, noggin, DMH1, connective tissue growth factor (CTGF), follistatin, sclerostin, DAN, chordin, gremlin, Cerberus 1, or inhibin, but is not limited thereto. The mitogenic growth factor is a protein that activates a mitogen-activated protein kinase (MAPK)-related signaling pathway, and may be, for example, hepatocyte growth factor (HGF), insulin-like growth factor (IGF), epidermal growth factor (EGF), fibroblast growth factor (FGF), keratinocyte growth factor (KGF), platelet-derived growth factor (PDGF), vascular endothelial growth factor (VEGF), neuregulin (NRG), or brain-derived neurotrophic factor (BDNF), but is not limited thereto.

Optionally, the preparation method according to the present invention may further comprise step e) of inducing self-assembly of epidermis in a product obtained in step d) using a fifth medium.

Optionally, the preparation method according to the present invention may further comprise step f) of maturing a product obtained in step e) using a sixth medium to prepare a skin organoid.

The first medium to the sixth medium may comprise matrigel, an antibiotic, an antioxidant, fetal bovine serum (FBS), GlutaMAX, N2, 2-mercaptoethanol (β-mercaptoethanol), and/or B27, which are commonly included in cell or organoid culture media in the art. The antibiotic may be, for example, penicillin, streptomycin, gentamicin, primocine, or normocin, but is not limited thereto. The antioxidant may be, for example, nicotinamide, 1-thioglycerol, valproic acid, or N-acetyl-L-cysteine, but is not limited thereto. The basal medium that may be used herein may be an animal cell culture medium commonly used in the art and may be, for example, RPMI1640, F-10, F-12, Dulbecco's Modified Eagle's Medium (DMEM), A-DMEM, advanced DMEM/F12, Minimal Essential Medium (MEM), Basal Medium Eagle (BME), α Minimal Essential Medium (αMEM), Glasgow's Minimal Essential Medium (GMEM), Iscove's Modified Dulbecco's Medium (IMEM), McCoy's 5A, Neurobasal medium, or a mixture thereof, but is not limited thereto.

Another aspect of the present invention provides a skin organoid prepared by the preparation method described above.

The skin organoid may be used for prevention or treatment of a skin disease. In addition, the skin organoid may be used in a method for evaluating efficacy of or screening an agent for preventing or treating a skin disease.

In the skin organoid according to the present invention, each term has the same meaning as described in the method for preparing a skin organoid unless specifically stated otherwise.

Another aspect of the present invention provides a pharmaceutical composition for preventing or treating a skin disease, comprising the skin organoid prepared by the preparation method described above.

In the pharmaceutical composition for preventing or treating a skin disease comprising the skin organoid according to the present invention, each term has the same meaning as described in the method for preparing a skin organoid unless specifically stated otherwise.

The skin organoid according to the present invention has a histological structure and function highly similar to those of human skin. Accordingly, when transplanted into skin, the skin organoid can regenerate damaged skin and thus may be used as an agent for preventing or treating a skin disease or symptom.

The pharmaceutical composition according to the present invention may be a cell therapeutic agent or a regenerative therapeutic agent.

As used herein, the term "cell therapeutic agent" refers to a pharmaceutical preparation prepared for purposes such as prevention, treatment, or diagnosis using living autologous, allogeneic, or xenogeneic cells. It may be used for regeneration of damaged or deficient cells or tissues and may be used to restore functions and morphology of the damaged or deficient cells or tissues.

The pharmaceutical composition according to the present invention may be administered to a subject in need of prevention or treatment of a skin disease by comprising the skin organoid in an effective amount.

The skin disease may be caused by skin damage or aging and may be, for example, atopic dermatitis, psoriasis, seborrheic dermatitis, urticaria, photosensitive dermatitis, acne, skin boils, furuncles, skin wrinkles, deterioration of skin elasticity, skin aging, wounds, skin pigmentation disorders, skin barrier damage, skin cancer, or alopecia, but is not limited thereto.

As used herein, the term "treatment" refers to any act by which symptoms of a skin disease are alleviated or cured by transplantation or administration of the skin organoid according to the present invention. In addition, as used herein, the term "prevention" refers to any act of suppressing or delaying symptoms of a skin disease by transplantation or administration of the skin organoid according to the present invention.

The effective amount may be a "therapeutically effective amount" or a "prophylactically effective amount". As used herein, the term "therapeutically effective amount" refers to any amount capable of reducing severity of symptoms of a disease, increasing frequency and duration of symptom-free periods of the disease, or preventing damage or disability caused by suffering from the disease when a drug or therapeutic agent is used alone or in combination with another therapeutic agent. As used herein, the term "prophylactically effective amount" refers to any amount capable of inhibiting occurrence or recurrence of a skin disease in a subject. The level of the effective amount may be determined depending on factors including severity, age, and sex of the subject, activity of the drug, sensitivity to the drug, administration time, administration route and excretion rate, treatment period, drugs used in combination therewith, and other factors well known in the medical field.

As used herein, the term "administration" refers to physically introducing a composition into a subject using any of various methods and delivery systems known to those skilled in the relevant art. Administration routes for the pharmaceutical composition of the present invention include, for example, submucosal, intramuscular, subcutaneous, intraperitoneal, spinal, or other parenteral administration routes, such as administration by injection or infusion, but are not limited thereto. In addition, the administration may be performed by a non-surgical administration method using a device such as a catheter. It may be performed by surgical administration methods such as transplantation or injection after incision of a diseased site. The number of administrations of the composition of the present invention may be, for example, a single administration, multiple administrations, or administration over one or more extended periods of time.

The pharmaceutical composition of the present invention may vary depending on age, sex, and body weight of a subject. Specifically, depending on symptoms of the subject, the pharmaceutical composition may be administered once or several times daily, or may be administered at intervals ranging from several days to several months. In addition, the dosage may be adjusted such that the skin organoids comprised in the pharmaceutical composition of the present invention are administered in an amount of 5×10⁵ cells/cm² to 5×10⁶ cells/cm², but is not limited thereto, and may be increased or decreased depending on administration route, severity of disease, sex, body weight, age, and the like.

The pharmaceutical composition of the present invention may further comprise suitable carriers, excipients, or diluents commonly used in preparation of pharmaceutical compositions. Examples of the carriers, excipients, and diluents that may be comprised in the composition include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil, but are not limited thereto.

As used herein, the term "subject" includes a human or any non-human animal. The non-human animal may be a vertebrate, such as a primate, dog, cow, horse, pig, or rodent, for example, a mouse, rat, or guinea pig. In the present specification, the term "subject" is used interchangeably with the terms "individual" and "patient."

The pharmaceutical composition of the present invention may be administered in combination with another therapeutic agent. In this case, the pharmaceutical composition of the present invention and the other therapeutic agent may be administered simultaneously, sequentially, or separately. The other therapeutic agent may be a drug such as a compound or protein having preventive, therapeutic, or ameliorative effects on a skin disease, but is not limited thereto.

In addition, the pharmaceutical composition of the present invention may be formulated for simultaneous, sequential, or separate administration with another therapeutic agent. For example, the skin organoid and the other therapeutic agent may be simultaneously administered in a single formulation, or may be simultaneously, sequentially, or separately administered in separate formulations. For simultaneous, sequential, or separate administration, the skin organoid comprised in the pharmaceutical composition of the present invention and the other therapeutic agent may each be separately formulated in different containers or may be jointly formulated in the same container. In addition, pharmaceutically effective amounts, administration times, administration intervals, administration routes, and treatment periods of the skin organoid comprised in the pharmaceutical composition of the present invention and the other therapeutic agent may be the same or different.

Another aspect of the present invention provides a method for preventing or treating a skin disease, comprising transplanting the skin organoid prepared according to the above-described method into a subject.

In the method for preventing or treating a skin disease according to the present invention, each term has the same meaning as described above unless specifically stated otherwise.

In the method for preventing or treating a skin disease according to the present invention, the skin organoid may be administered to a subject simultaneously, sequentially, or separately with another therapeutic agent.

The term "simultaneous" administration refers to administration of the skin organoid and another therapeutic agent at one time in a single formulation, or administration of the skin organoid and another therapeutic agent at one time in separate formulations, in which case administration routes of the skin organoid and the other therapeutic agent may be different from each other. The term "sequential" administration refers to administration of the skin organoid and another therapeutic agent in a relatively consecutive manner, allowing the minimum possible time required for an administration interval. The term "separate" administration refers to administration of the skin organoid and another therapeutic agent at predetermined time intervals. Administration methods of the skin organoid and the other therapeutic agent may be appropriately selected by a physician or expert in the art in consideration of therapeutic efficacy, side effects, and the like in a subject.

Another aspect of the present invention provides a method for evaluating efficacy of or screening a candidate material for prevention or treatment of a skin disease or a cosmetic, comprising treating the skin organoid prepared according to the above-described method with the candidate material for prevention or treatment of a skin disease or the cosmetic.

In the method for evaluating efficacy of or screening a material for prevention or treatment of a skin disease or a cosmetic according to the present invention, each term has the same meaning as described above unless specifically stated otherwise.

As used herein, the term "cosmetic" is not particularly limited as long as it is intended to improve skin condition and may be, for example, a preparation developed for purposes such as skin moisturization, enhancement of skin elasticity, or improvement of skin wrinkles.

As used herein, the term "candidate material" refers to a material expected to treat, prevent, or ameliorate a skin disease. Specifically, any material expected to directly or indirectly alleviate or improve a skin disease may be used without limitation, and includes candidate materials such as compounds, genes, and proteins.

Treatment of the candidate material may be performed using methods known in the art. Specifically, the candidate material may be treated by co-culturing it with the skin organoid or by transplanting or administering it into an in vivo system comprising the skin organoid, but is not limited thereto, and those skilled in the art may use any method suitable for the purpose of the present invention.

The method for evaluating efficacy and the screening method according to the present invention may comprise step a) of treating the organoid of the present invention with a candidate material and step b) of comparing the candidate material-treated skin organoid with a control group.

As used herein, the term "control group" refers to a skin organoid not treated with the candidate material or a skin organoid not treated with a material for prevention or treatment of a skin disease. The control group may also include a positive control group. The control group may be used to compare results with those of the candidate material-treated skin organoid.

The skin organoid prepared according to the present invention has high histological similarity to human skin tissue, may comprise hair follicles, and in particular, can regenerate damaged skin tissue when transplanted in vivo.

Accordingly, the method for preparing a skin organoid according to the present invention may be used to prepare a skin organoid having morphology and function similar to those of living tissue, and the prepared skin organoid may be useful as a regenerative therapeutic material for a skin disease or may be advantageously used in a method for evaluating efficacy of or screening a therapeutic material for a skin disease.

### [Brief Description of Figures]

FIG. 1 shows images of skin organoids prepared using induced pluripotent stem cells (scale bar: 500 µm). D1, D2, D4, D6, and D8 indicate 1 day, 2 days, 4 days, 6 days, and 8 days, respectively, from the start date of culture.
FIG. 2 shows images of skin organoids prepared using embryonic stem cells (scale bar: 500 µm). D1, D2, D4, D6, D8, and D40 indicate 1 day, 2 days, 4 days, 6 days, 8 days, and 40 days, respectively, from the start date of culture.
FIG. 3 shows images of skin organoids prepared using induced pluripotent stem cells (n=3).
FIG. 4 shows images of skin organoids prepared using embryonic stem cells (n=3).
FIGs. 5a and 5b show images illustrating histochemical staining results of DAPI and AP2α protein, which is an ectoderm expression marker, in skin organoids harvested 4 days after initiation of culture using induced pluripotent stem cells in order to confirm similarity of the skin organoids to skin tissue.
FIG. 5b shows images illustrating histochemical staining results of DAPI, E-cadherin (ECAD), which is an ectoderm expression marker, and SOX10 protein, which is a cranial neural crest cell expression marker, in skin organoids harvested 4 days after initiation of culture using induced pluripotent stem cells in order to confirm similarity of the skin organoids to skin tissue.
FIG. 6a shows images illustrating histochemical staining results of DAPI and AP2α protein, which is an ectoderm expression marker, in skin organoids harvested 4 days after initiation of culture using embryonic stem cells in order to confirm similarity of the skin organoids to skin tissue.
FIG. 6b shows images illustrating histochemical staining results of DAPI, E-cadherin, which is an ectoderm expression marker, and SOX10 protein, which is a cranial neural crest cell expression marker, in skin organoids harvested 4 days after initiation of culture using embryonic stem cells in order to confirm similarity of the skin organoids to skin tissue.
FIG. 7a shows images illustrating histochemical staining results of DAPI and AP2α protein, which is an ectoderm expression marker, in skin organoids harvested 8 days after initiation of culture using induced pluripotent stem cells in order to confirm similarity of the skin organoids to skin tissue.
FIG. 7b shows images illustrating histochemical staining results of DAPI, E-cadherin, which is an ectoderm expression marker, and SOX10 protein, which is a cranial neural crest cell expression marker, in skin organoids harvested 8 days after initiation of culture using induced pluripotent stem cells in order to confirm similarity of the skin organoids to skin tissue.
FIG. 8a shows images illustrating histochemical staining results of DAPI and AP2α protein, which is an ectoderm expression marker, in skin organoids harvested 8 days after initiation of culture using embryonic stem cells in order to confirm similarity of the skin organoids to skin tissue.
FIG. 8b shows images illustrating histochemical staining results of DAPI, E-cadherin, which is an ectoderm expression marker, and SOX10 protein, which is a cranial neural crest cell expression marker, in skin organoids harvested 8 days after initiation of culture using embryonic stem cells in order to confirm similarity of the skin organoids to skin tissue.

### [Examples]

Hereinafter, the present invention will be described in more detail through examples. These examples are provided to more specifically describe the present invention, and the scope of the present invention is not limited thereto.

### Preparation Example

Embryoid bodies (EBs) were formed using induced pluripotent stem cells (iPSCs) or embryonic stem cells (ESCs). Specifically, hPSCs such as iPSCs and ESCs were dissociated into single cells using a cell dissociation solution, followed by counting the number of cells and seeding 3×10³ to 5×10³ cells per well in a 96-well low-attachment plate to form embryoid bodies (EBs). Embryoid bodies, which are aggregates of hPSCs, are commonly used to efficiently induce differentiation. Although EBs are initially composed mostly of undifferentiated hPSCs, the undifferentiated cells are gradually differentiated into surface ectoderm (SE) cells or cranial neural crest (CNC) cells depending on culture conditions.

The present invention is characterized in that a first medium is used until differentiation of surface ectoderm is induced from pluripotent stem cells, such as induced pluripotent stem cells and embryonic stem cells, or embryoid bodies, and a second medium is used until differentiation of cranial neural crest (CNC) cells is induced from pluripotent stem cells, such as induced pluripotent stem cells and embryonic stem cells, or embryoid bodies, wherein BMP comprised in the first medium is present at a higher concentration than BMP comprised in the second medium.

In the present Preparation Example, the content of BMP comprised in the first medium and the content of BMP comprised in the second medium were differently set. Induced pluripotent stem cells were used in the Preparation Examples described in Table 1 below, whereas embryonic stem cells were used in the Preparation Examples described in Table 2 below. In Preparation Examples 1 and 5 and Preparation Examples 6 and 10, BMP concentrations in the first medium and the second medium are not different from each other. Preparation Examples 3, 4, and 11 and Preparation Examples 8, 9, and 12 have a downward BMP concentration gradient from the first medium to the second medium.

**[Table 1]**

| | Content of BMP comprised in the first medium (ng/mL) | Content of BMP comprised in the second medium (ng/mL) |
|---|---|---|
| Preparation Example 1 | 2.5 | 2.5 |
| Preparation Example 2 | 25 | 0 |
| Preparation Example 3 | 25 | 1 |
| Preparation Example 4 | 25 | 1.5 |
| Preparation Example 5 | 25 | 25 |
| Preparation Example 11 | 10 | 1 |

**[Table 2]**

| | Content of BMP comprised in the first medium (ng/mL) | Content of BMP comprised in the second medium (ng/mL) |
|---|---|---|
| Preparation Example 6 | 2.5 | 2.5 |
| Preparation Example 7 | 25 | 0 |
| Preparation Example 8 | 25 | 1 |
| Preparation Example 9 | 25 | 1.5 |
| Preparation Example 10 | 25 | 25 |
| Preparation Example 12 | 10 | 1 |

DMEM/F12 medium was used as a basal medium for the first medium, to which BMP4 as a BMP and SB431542 as a TGFβ inhibitor were added. The day on which culturing of embryoid bodies (EBs) in the first medium was initiated was designated as D0. After culturing for 2 days, differentiation into surface ectoderm was confirmed. Subsequently, on culture day 2 (D2), the medium was replaced with a second medium, and after culturing for 2 days, differentiation of undifferentiated cells into cranial neural crest (CNC) cells was confirmed.

Thereafter, LDN193189 as a BMP inhibitor was added to the medium, followed by additional culturing for 2 days. Subsequently, on culture day 6 (D6), for maturation of skin organoids, the medium was replaced with a mixed medium of Advanced DMEM/F12 medium and Neurobasal medium supplemented with GlutaMAX supplement, B-27 supplement, 2-mercaptoethanol and normocin, which are commonly used for organoid culture. Skin organoids were each harvested and photographed at 2 days after medium replacement on D8 (D10), 22 days after medium replacement on D8 (D30), and 42 days after medium replacement on D8 (D50).

### Experimental Example 1

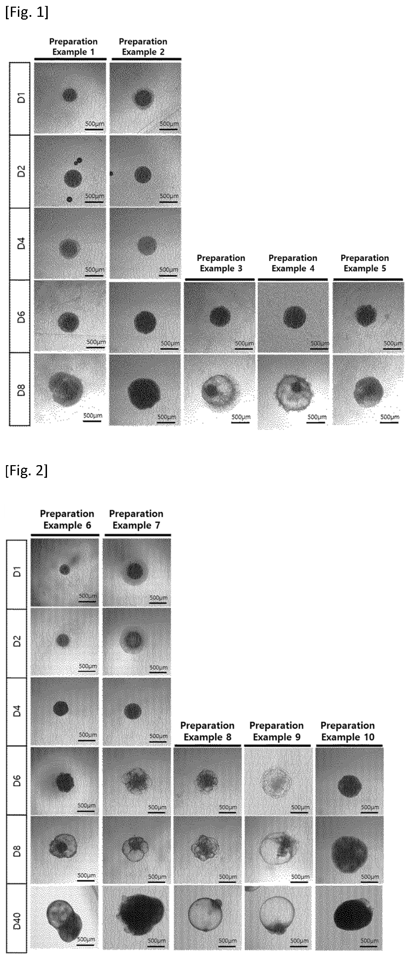

FIG. 1 shows photographs taken using a phase-contrast microscope on D1, D2, D4, D6, and D8 when the same iPSC#1 cells were respectively used according to the conditions described in Table 1. In addition, FIG. 2 shows photographs taken using a phase-contrast microscope on D1, D2, D4, D6, D8, and D40 when the same hESC#1 cells were respectively used according to the conditions described in Table 2.

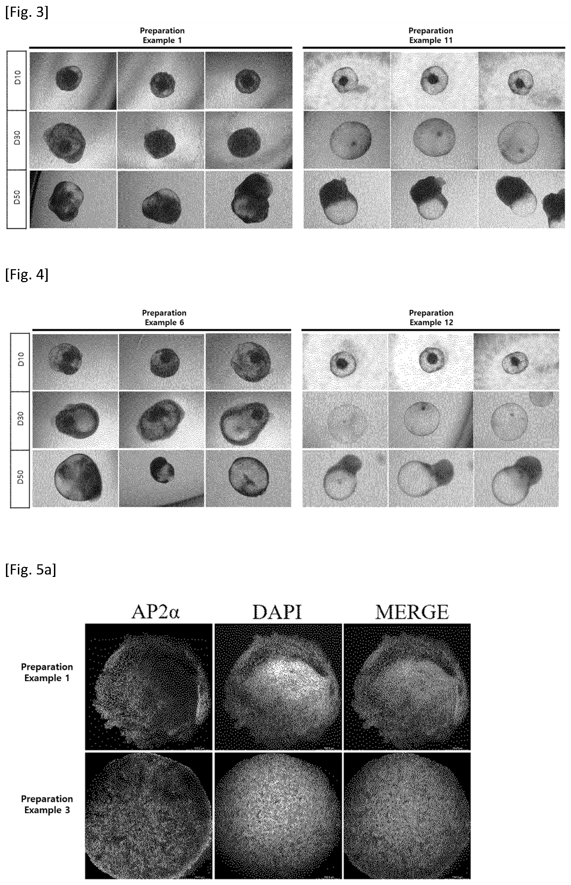

FIG. 3 shows photographs taken using a phase-contrast microscope on D10, D30, and D50 when iPSC#2 cells were respectively used according to the conditions of Preparation Examples 1 and 11, showing results of three independent repeated experiments (n=3). In addition, FIG. 4 shows photographs taken using a phase-contrast microscope on D10, D30, and D50 when ESC#2 cells were respectively used according to the conditions of Preparation Examples 6 and 12, showing results of three independent repeated experiments (n=3).

As can be seen in FIGs. 1 and 3, Preparation Examples 3, 4, and 12, which employed a downward BMP concentration gradient, showed effective formation of cyst structures that are morphologically important in an early stage of skin differentiation, indicating uniform induction of skin organoid differentiation. In contrast, in the Preparation Examples 1, 2, and 5, cyst structures were not formed, indicating that induction of an early stage of skin differentiation was not effectively achieved. As described above, in Preparation Examples in which differentiation was induced without a concentration gradient, failure rates of skin organoid differentiation at an early stage were high, and even when skin organoids were differentiated, they were observed to be morphologically irregular, non-uniform, or contracted.

As can be seen in FIGs. 2 and 4, Preparation Examples 6, 7, 8, 9, and 12 showed effective formation of cyst structures that are morphologically important in an early stage of skin differentiation, whereas in Preparation Example 10, cyst structures were not formed, indicating that induction of an early stage of skin differentiation was not effectively achieved. In addition, in Preparation Examples 6 and 7, although cyst formation was induced during an early stage of skin differentiation, problems were observed in that induction of skin layers was not properly achieved during a skin maturation process or skin organoids having non-uniform sizes and shapes were formed. In contrast, in Preparation Examples 8, 9, and 12, which employed a downward BMP concentration gradient, induction of skin layers was normally achieved during the maturation process, and organoids having uniform sizes and shapes were observed to be formed.

Through the above experiments, it was demonstrated that, in an early stage of skin organoid differentiation, differentiation into surface ectoderm, which plays an important role in differentiation of the epidermal layer of skin, and cranial neural crest (CNC) cells, which play an important role in differentiation of the dermal layer, could be efficiently induced through a BMP concentration gradient between the first medium and the second medium, and that uniform and mature skin organoids could be prepared.

### Experimental Example 2

In this Experimental Example, immunohistochemical analysis was performed to confirm whether the previously prepared human skin organoids are histologically similar to actual human skin.

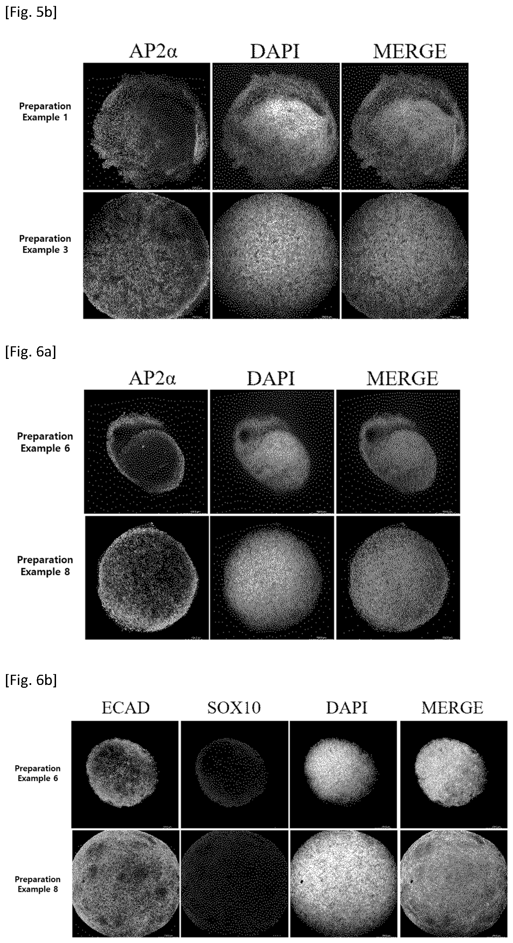

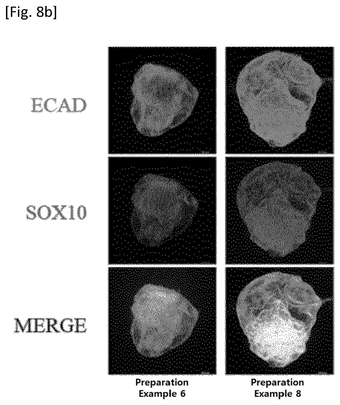

Specifically, immunofluorescence staining was performed on skin organoids harvested on day 4 (D4) and day 8 (D8) from the culture initiation date using DAPI for staining nuclei of cells; an antibody against E-cadherin (ECAD) protein as a surface ectoderm expression marker; and antibodies against SOX10 and AP2α proteins as cranial neural crest cell expression markers. The results are shown in FIGs. 5 to 8.

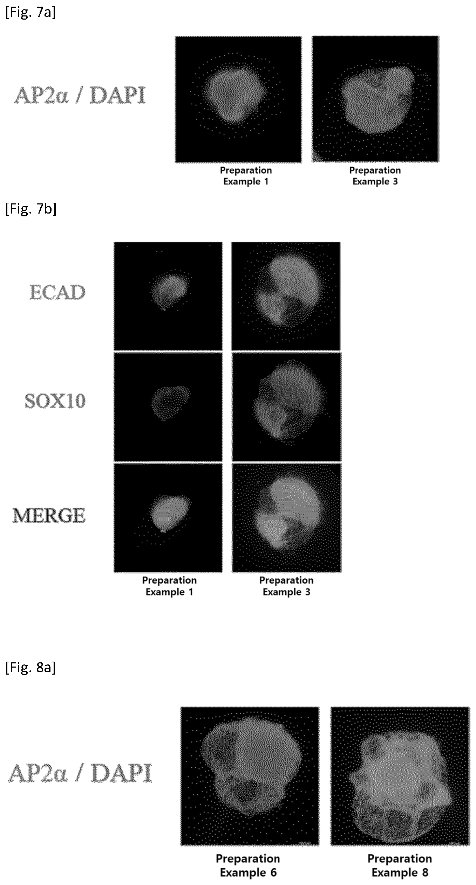

FIGs. 5a to 6b are photographs obtained by harvesting and staining skin organoids (Preparation Examples 1, 3, 6, and 8) on day 4 (D4) from the culture initiation date, and FIGs. 7a to 8b are photographs obtained by harvesting and staining skin organoids (Preparation Examples 1, 3, 6, and 8) on day 8 (D8) from the culture initiation date.

As a result, compared to Preparation Example 1 using induced pluripotent stem cells without a BMP concentration gradient, Preparation Example 3 using a higher concentration of BMP in the first medium through a BMP concentration gradient showed that surface ectoderm was more effectively induced. In addition, compared to Preparation Example 1 without a BMP concentration gradient, Preparation Example 3 employing a concentration gradient showed that cranial neural crest cells were more effectively induced.

Similar to the results obtained using induced pluripotent stem cells, compared to Preparation Example 6 using embryonic stem cells without a BMP concentration gradient, Preparation Example 8 using a higher concentration of BMP in the first medium through a BMP concentration gradient showed that ectoderm was more effectively induced. In addition, compared to Preparation Example 6 without a BMP concentration gradient, Preparation Example 8 employing a concentration gradient showed that cranial neural crest cells were more effectively induced.

The above results demonstrate that when the BMP concentration comprised in the second medium is lower than the BMP concentration comprised in the first medium, that is, when a downward BMP concentration gradient is formed, surface ectoderm and cranial neural crest cells are each significantly more effectively differentiated, and accordingly, multilayered structures of the prepared skin organoids are more effectively formed. These results suggest that the method of the present invention is efficient for preparation of skin organoids and capable of enhancing similarity to skin tissue.

## Claims

1. A method for preparing a skin organoid, comprising:
a) culturing pluripotent stem cells or embryoid bodies (EBs) in a first medium comprising BMP to differentiate into surface ectoderm; and
b) culturing a product of step a) in a second medium comprising BMP to differentiate into cranial neural crest cells,
wherein a BMP concentration in the second medium is lower than a BMP concentration in the first medium.

2. The method of claim 1, wherein the pluripotent stem cells are embryonic stem cells or induced pluripotent stem cells.

3. The method of claim 1, wherein the BMP is selected from the group consisting of BMP2, BMP3, BMP4, BMP6, BMP7, BMP8, BMP9, BMP10, BMP11, and BMP15.

4. The method of claim 1, wherein the first medium and the second medium further comprise a transforming growth factor β (TGFβ) inhibitor.

5. The method of claim 4, wherein the TGFβ inhibitor is selected from the group consisting of A83-01, SB-431542, SB-505124, SB-525334, SD-208, LY-364947, and SJN-2511.

6. A skin organoid prepared according to the method of claim 1, wherein the skin organoid comprises a multilayered structure including an epidermal layer, a dermal layer, and a subcutaneous tissue layer.

7. The skin organoid of claim 6, wherein the skin organoid comprises a hair follicle.

8. The skin organoid of claim 6, wherein the skin organoid is used for prevention or treatment of a skin disease.

9. The skin organoid of claim 8, wherein the skin disease is one or more selected from the group consisting of atopic dermatitis, psoriasis, seborrheic dermatitis, urticaria, photosensitive dermatitis, acne, skin boils, furuncles, skin wrinkles, deterioration of skin elasticity, skin aging, wound alleviation, skin pigmentation disorders, skin barrier damage, skin cancer, and alopecia.

10. The skin organoid of claim 6, wherein the skin organoid is used for evaluating efficacy of a cosmetic or an agent for treating or preventing a skin disease.

11. The skin organoid of claim 6, wherein the skin organoid is used for screening a cosmetic or an agent for treating or preventing a skin disease.

12. A pharmaceutical composition for treating or preventing a skin disease, comprising the skin organoid of claim 6.

13. The pharmaceutical composition of claim 12, wherein the skin disease is one or more selected from the group consisting of atopic dermatitis, psoriasis, seborrheic dermatitis, urticaria, photosensitive dermatitis, acne, skin boils, furuncles, skin wrinkles, deterioration of skin elasticity, skin aging, wound alleviation, skin pigmentation disorders, skin barrier damage, skin cancer, and alopecia.

14. A method for evaluating efficacy of a cosmetic or an agent for treating or preventing a skin disease, using the skin organoid of claim 6.

15. A method for screening a cosmetic or an agent for treating or preventing a skin disease, using the skin organoid of claim 6.
